# EUROPÄISCHE PATENTSCHRIFT

(11) **EP 0 820 593 B1**
(45) Veröffentlichungstag und Bekanntmachung des Hinweises auf die Patenterteilung: **22.12.1999**
(21) Anmeldenummer: 96911977.5
(22) Anmeldetag: 30.03.1996
(51) Int. Cl.: G01N 33/543, G01N 27/414, G01N 27/327

(54) **MESSEINRICHTUNG**
MEASURING DEVICE
DISPOSITIF DE MESURE

(30) Priorität: 04.04.1995 DE 19512117
(43) Veröffentlichungstag der Anmeldung: 28.01.1998
(73) Patentinhaber: Micronas Intermetall GmbH, 79108 Freiburg (DE)
(72) Erfinder: WOLF, Bernhard, D-79252 Stegen (DE); EHRET, Ralf, D-79291 Merdingen (DE); SIEBEN, Ulrich, D-79276 Reute (DE); BAUMANN, Werner, D-77815 Bühl-Altschweier (DE)
(74) Vertreter: Schmitt, Hans, Dipl.-Ing.
(86) Internationale Anmeldenummer: EP9601409
(87) Internationale Veröffentlichungsnummer: WO9631774

(56) Entgegenhaltungen:
- EP-A- 0 127 438
- EP-A- 0 402 917
- WO-A-88/09808
- WO-A-93/08464
- WO-A-93/22678
- Kraus et al, Bioscope 1993, Nr. 1, S. 24 - 33

## Beschreibung

Die Erfindung bezieht sich auf eine Meßeinrichtung nach dem Oberbegriff von Anspruch 1.

Aus der Druckschrift "Kraus et al, BIOSCOPE 1993, Nr. 1, Seite 24 bis 33" ist bereits eine Meßeinrichtung nach dem Oberbegriff von Anspruch 1 bekannt. Obwohl sich diese Meßeinrichtung insbesondere wegen ihrer schnellen Ansprechzeit und der Möglichkeit selektive Messungen in dem Analyt durchzuführen in der Praxis bewährt hat, ist die funktionserhaltende Immobilisierung der Rezeptor- und/oder Targetzellen auf der physikalischen Komponente des Sensors äußerst kritisch. Eine Möglichkeit, die Rezeptor- oder Targetzellen mit der Meßstruktur der Meßeinrichtung in Kontakt zu bringen, besteht beispielsweise darin, die Rezeptor- und/oder Targetzellen mit einem Gel unverrückbar an der Meßstruktur zu fixieren.

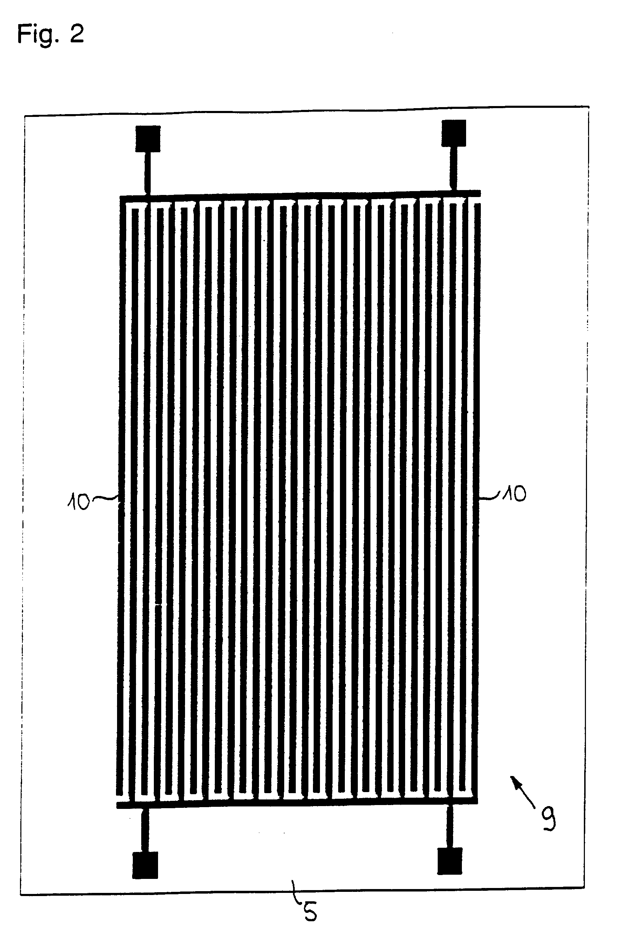

Gemäß einer anderen Alternative werden die Zellen direkt auf dem Gate eines FET immobilisiert (siehe Abb. 2b).

Man kennt auch bereits eine Meßeinrichtung, bei der die zu untersuchenden Zellen in einem biologischen Puffermedium enthalten sind und bei der diesem Puffermedium ein Hilfsreagenz zugesetzt ist, das beim Vorhandensein bestimmter chemischer Komponenten eine Färbung des Puffermediums bewirkt. So kann beispielsweise ein Calcium-Farbstoff zum Nachweis von Calcium-Ionen in dem Puffermedium enthalten sein. Zur Messung einer durch die nachzuweisende Substanz bewirkte Farbveränderung weist die vorbekannte Meßeinrichtung einen optischen Sensor und eine Lichtquelle zum Durchleuchten des Puffermediums auf. Ein Nachteil dieser Meßeinrichtung besteht vor allem darin, daß sie eine gewisse Größe aufweist und daher für bestimmte Messungen nicht flexibel genug einsetzbar ist. Außerdem ist die Messung nicht rückwirkungsfrei, da die benötigten Hilfsreagenzien zum Teil toxisch sind und die zu untersuchenden Zellen oder die zellulären Targets beeinflussen. Auch die hohen Photonendichten können zu Veränderungen in dem Meßsystem führen.

Außerdem sind Sensoren auf enzymatischer Basis oder mit mikrobieller Struktur bekannt. Damit ist jedoch nur eine Einzelsignalanalyse möglich, d.h. diese Sensoren messen immer nur einen Analyten. Außerdem sind solche Sensoren störanfällig, da die Messung eines Störsignales keine sichere Analyterkennung zuläßt.

Es besteht daher die Aufgabe, eine Meßeinrichtung der eingangs genannten Art zu schaffen, die eine kompakte Baugröße aufweist, bei der die Beeinflussung des zu untersuchenden Mediums durch die Meßeinrichtung vermindert ist und die neben einer selektiven Auswertung auch eine hohe Meßempfindlichkeit aufweist.

Die Lösung dieser Aufgabe besteht darin, daß zwischen den Rezeptor- und/oder Targetzellen und der Meßstruktur eine strukturierte, mikroporöse Zwischenschicht vorgesehen ist, welche die Target- und/oder Rezeptorzellen als Nachbarn zur Adhärenz akzeptieren.

Ein solcher Sensor, der prinzipiell aus einem eingangsseitigen, Sensorteil, analog einem elektrischen Diskriminator und einem durch die elektrische oder elektronische Meßstruktur gebildeten Transducer besteht, ermöglicht Messungen mit sehr hoher Meßempfindlichkeit. Dabei lassen tierische oder pflanzliche Zellen als Bestandteil des Sensors, die spontan adhärent oder zur Adhärenz mit der Meßstruktur gebracht wurden, eine hochsensible, dynamische Stofferkennung zu, da als Diskriminatoreinheit die parallelen Signalverarbeitungsmechanismen der Zellen ausgenützt werden.
Ein weiterer Vorteil stellt die Tatsache dar, daß Zellmembrangebundene Rezeptoren nach Beladung mit einem Analyten durch die Zellen sofort recycelt werden und ein neuer Rezeptor an die Zelloberfläche transportiert wird. Dieser Effekt kann durch biotechnologische Maßnahmen gezielt optimiert werden (z.B. durch transgene Rezeptoren). Im Vergleich zu konventionellen und auch mikrobiellen Sensoren werden damit hochdynamische und durch den internen Signalverstärkungsapparat der Zellen, hochsensitive Sensorstrukturen möglich.
Bei der erfindungsgemäßen Meßeinrichtung wirkt also der Sensor mit Target- oder Rezeptorzellen zusammen, die unmittelbar auf der Meßstruktur angeordnet sind und bei Berührung mit dem Analyten oder dem zu untersuchenden Medium durch bestimmte, darin enthaltene Substanzen beeinflußt werden. Die dadurch bewirkten Veränderungen in den Target- oder Rezeptorzellen sind mit dem Sensor direkt meßbar, so daß das von dem Sensor abgegebene elektrische Signal Rückschlüsse auf die in dem Analyten enthaltenen Substanzen und/oder Zellen ermöglicht. Dabei können abhängig von den speziellen Eigenschaften der mit dem Sensor gekoppelten funktionsspezifischen Rezeptor- und/oder Targetzellen unterschiedlichste Substanzen gemessen oder nachgewiesen werden. So können beispielsweise Rezeptorzellen zum Nachweis von Opiaten, Atracin, Steroiden oder Östrogenen vorgesehen sein. Mittels spezieller Targetzellen können biologische (z.B. toxische), chemische (z.B. Schwermetalle etc.) und physikalische (z.B. Strahlung) Komponenten detektiert werden. Da die erfindungsgemäße Meßvorrichtung eine sehr geringe Baugröße aufweist, ist sie besonders gut handhabbar und kann auch an schwer zugänglichen Stellen eingesetzt werden. Außerdem wird für die Messung nur eine entsprechend geringe Menge des zu untersuchenden Mediums benötigt. Da der zu untersuchenden Substanz bzw. dem Analyten keine Hilfsreagenzien zugesetzt werden müssen, arbeitet die Meßvorrichtung praktisch rückwirkungsfrei. Die vorgesehene Zwischenschicht ist insbesondere eine makromolekulare poröse Schicht, die einerseits die Zellen zur Adhäsion bringt und andererseits in der Porengröße so bemessen ist, daß sie für bestimmte Ionen, Moleküle oder Zellbereiche durchlässig ist. Als Zwischenschicht kann beispielsweise auch eine gesputterte oder auf die Meßstruktur aufgebrachte SiO₂-Schicht, eine Al₂O₃-Schicht oder eine Ta₂O₅-Schicht vorgesehen sein. Durch die strukturierte Zwischenschicht wird die elektronische Meßstruktur so konditioniert, daß die Target- oder Rezeptorzellen die Meßstruktur als Nachbar akzeptieren und sich an diese besser anlagern. Die Porosität der Zwischenschicht ermöglicht dabei, daß die zu messenden Ionen, Moleküle oder Zellbereiche der Target- oder Rezeptorzellen zu den elektrisch aktiven Bereichen der Meßstruktur gelangen können.

Besonders vorteilhaft ist, wenn auf einem gemeinsamen Substrat mehrere Sensoren mit Meßstrukturen insbesondere als Sensorarray angeordnet sind.
Ein solches Sensorarray kann als integrierte Schaltung besonders kostengünstig hergestellt werden und ermöglicht auf engstem Raum die Messung unterschiedlichster chemischer oder biologischer Substanzen.
Es besteht dabei die Möglichkeit, daß auf einem gemeinsamen Substrat mehrere Sensoren mit gleichen Meßstrukturen insbesondere als Sensorarray angeordnet sind.
Andererseits können auf einem gemeinsamen Substrat auch mehrere Sensoren mit unterschiedlichen Meßstrukturen insbesondere als Sensorarray angeordnet sind.
Weiterhin können auf einem gemeinsamen Substrat mehrere zellbehaftete Meßstrukturen und zusätzlich zellfreie Meßstrukturen vorgesehen sind. Zelluläre Sensorstrukturen und zellfreie Meßstrukturen liefern ein paralleles Datenmuster, das stoff- und funktionsspezifische Sensorleistungen ermöglicht.

Zweckmäßigerweise sind die Meßausgänge der auf einem gemeinsamen Substrat angeordneten Sensoren mit einer insbesondere auf dem Substrat integriert angeordneten Steuer- und Auswerteeinrichtung verbunden. In der integrierten Steuer- und Auswerteeinrichtung kann beispielsweise eine Vorverarbeitung der Meßwerte vorgenommen werden. Die Auswerteelektronik erlaubt auch ein stoff- und funktionsspezifisches Training des Sensors.

Eine noch flexibleren Nutzung der auf dem Substrat befindlichen Sensoren kann dadurch erreicht werden, daß auf dem Substrat mit dem Sensorarray als Steuer- und Auswerteeinrichtung ein Multiplexer, ein AD/DA-Wandler mit Sensoransteuerung, ein Mikroprozessor sowie eine IO-Einheit angeordnet sind. Der Mikroprozessor kann dann Offsetspannungen und Temperaturdrifte der Sensoren kompensieren und gegebenenfalls auch die Verarbeitung oder Auswertung der Meßwerte übernehmen. Der Multiplexer ermöglicht in Verbindung mit der IO-Einheit sowie dem AD/DA-Wandler die Übertragung der mit einer Vielzahl von Sensoren gleichzeitig ermittelten Meßwerte über einen Datenbus oder über eine gemeinsame Datenleitung.
Eine bevorzugte Ausführungsform der Erfindung sieht vor, daß auf dem die Meßstruktur aufweisenden Substrat innerhalb des Meßbereiches oder benachbart zu diesem wenigstens eine Reizelektrode für die Rezeptor- oder Targetzellen angeordnet ist. Die Target- oder Rezeptorzellen können dann mittels der Reizelektrode gezielt angeregt und zur spontanen Abgabe einer mit dem Sensor zu detektierenden physiologischen Substanz veranlaßt werden.
Besonders günstig ist, wenn das Substrat ein Halbleitersubstrat ist und wenn als Meßstruktur wenigstens ein Feldeffekttransistor, insbesondere ein ISFET vorgesehen ist. Zwischen dem Gate und den Target- oder Rezeptorzellen ist eine Zwischenschicht, beispielsweise eine SiO₂-Schicht für eine Potentialmessung mit erwünschter Querempfindlichkeit für pH oder aber auch eine Aluminiumoxid- oder Tantalpentoxid-Schicht für protonenspezifische Messungen vorgesehen. Ferner kann auch eine Festkörpermembran, beispielsweise eine dünne Glasmembran auf den Gate abgeschieden sein.

Eine andere, vorteilhafte Ausführungsform sieht als Meßstruktur wenigstens einen Interdigitalkondensator, der vorzugsweise paarig angeordnete Elektroden beispielsweise in Kamm- oder Mäanderstruktur aufweist, vor. Dabei ist der Abstand der Kamm- bzw. Kondensatorelektroden an den Durchmesser der Target- oder Rezeptorzellen angepaßt, so daß diese mit unterschiedliche gepolten Kondensatorelektroden in Berührung stehen können. Ein Interdigitalkondensator als Meßstruktur eignet sich besonders zur Messung von Formveränderungen der Target- oder Rezeptorzellen, die beispielsweise bei Targetzellen durch die Anwesenheit von Schwermetallen oder bei transgenen Zellen durch den Kontakt mit einem bestimmten Rezeptor hervorgerufen sein können. Außerdem können mit Interdigitalkondensatoren Impedanz- oder Kapazitätsänderungen der Zellmembran gemessen werden. Auch Antikörper, die sich an den Target- oder Rezeptorzellen anlagern, können mit einer Interdigitalstruktur nachgewiesen werden, da sie die Dielektrizitätskonstante im Bereich der Interdigitalstruktur verändern. Zur Messung von Kapazitätsänderungen ist vorzugsweise zwischen den Elektroden des Interdigitalkondensators und den Target- oder Rezeptorzellen eine dünne Isolationsschicht vorgesehen.

Zweckmäßigerweise sind für die Meßeinrichtung mehrere, vorzugsweise unterschiedlich große Interdigitalkondensatoren vorgesehen. Die einzelnen Sensoren weisen dann eine unterschiedliche Empfindlichkeit auf, so daß die Meßeinrichtung einen größeren Meßbereich abdeckt und in den einzelnen Meßbereichen eine höhere Auflösung ermöglicht. Die Empfindlichkeit der Meßeinrichtung kann auch dadurch verbessert werden, daß der Analyt vor oder während der Messung relativ zu den Target- oder Rezeptorzellen beispielsweise durch Umrühren des Analyten oder durch Schütteln der Meßeinrichtung bewegt wird.

Vorteilhaft ist, wenn in wenigstens einem isolierten Zwischenraum der Elektroden des Interdigitalkondensators eine elektrochemosensitive Schicht vorgesehen ist. Der Sensor ist dann zum Nachweis bestimmter, von den Target- oder Rezeptorzellen abgeschiedenen physiologischen Substanzen, beispielsweise Sauerstoff oder komplexe Gase besser geeignet. Hierzu können in den Zwischenräumen elektroaktive Substanzen aufgebracht oder in keramischen Schwämmen verpackt sein.

Bei einer anderen Ausführungsform sind zwischen den Elektroden des Interdigitalkondensators Lichtleiter vorgesehen und zur Aufnahme und zum Nachweis des den jeweiligen Lichtleiter durchlaufenden Lichts sind Lichtdetektoren im Substrat angeordnet. Die Meßeinrichtung stellt dann zusätzliche Informationen über beispielsweise von den Target- oder Rezeptorzellen abgegebenes Streulicht zur Verfügung, die einen Rückschluß auf die Vitalität der Zellen ermöglichen. In vorteilhafter Weise kann somit mit Hilfe der Lichtdetektoren ein Selbsttest der Meßeinrichtung durchgeführt werden.

Eine noch exaktere Kontrolle der mit den Meßstrukturen in Kontakt stehenden Target- oder Rezeptorzellen wird dadurch ermöglicht, daß in das Substrat CCD-Sensoren, insbesondere in Form einer CCD-Zeile oder eines CCD-Arrays integriert sind. Dadurch wird eine noch höhere Auflösung bei der optischen Messung erreicht, so daß es insbesondere auch möglich ist, morphologische Veränderungen einzelner oder mehrerer, in bestimmten Bereichen der Meßstruktur angeordneter Zellen, zu überwachen.

Zweckmäßigerweise ist auf dem die Meßstruktur aufweisenden Substrat außerhalb des für die Messung vorgesehenen Substratbereiches wenigstens ein Referenzelement mit gleichem oder ähnlichem Aufbau wie dem der Meßstruktur angeordnet, insbesondere bei einem Feldeffekttransistor als Meßstruktur ein Feldeffekttransistor mit Gate. Mit solchen Referenzelementen können beispielsweise Temperaturdrifte oder Offsetspannungen der für die Messung vorgesehenen Meßstrukturen kompensiert werden.

Vorteilhaft ist, wenn auf dem die Meßstruktur aufweisenden Substrat wenigstens ein Temperaturmeßsensor, insbesondere eine Temperaturmeßdiode angeordnet ist. Die Temperaturabhängigkeit der biologischen Aktivität der Target- oder Referenzzellen kann dann bei der Messung berücksichtigt und gegebenenfalls kompensiert werden.

Eine Ausführungsform der Erfindung sieht vor, daß die Rezeptorzellen in einer Gelstruktur angeordnet sind oder von dieser überdeckt sind. Durch die Gelstruktur können die zum Sensor gehörenden Zellen zur Adhärenz an der Meßstruktur gebracht werden und so unmittelbar mit der Meßstruktur des Sensors in Kontakt gebracht und mit dieser immobil verbunden werden. Die Gelstruktur wirkt auch als Feuchtigkeits- und Nahrungsspeicher für die Target- oder Rezeptorzellen, so daß diese über einen längeren Zeitraum hinweg am Leben erhalten werden können. Die Meßvorrichtung ermöglicht dadurch beispielsweise über einen Zeitraum von etwa 10 Tagen eine ununterbrochene on-line-Signalgewinnung für eine in dem Analyten enthaltene biologische oder chemische Komponente. Ein Vorteil der Gelstruktur besteht auch darin, daß die Maschenweite der Gelstruktur an den Durchmesser der zu detektierenden Ionen, Moleküle oder Zellbereiche angepaßt werden kann, so daß Ionen, Moleküle oder Zellbereiche, die einen größeren Durchmesser als die Maschenweite oder die entsprechende Ladungsprofile aufweisen, erst gar nicht bis zu den Target- oder Rezeptorzellen vordringen können.

Eine Weiterbildung der Erfindung sieht vor, daß die elektrische oder elektronische Meßstruktur Teil einer Wandung eines Aufnahmebehältnisses für das zu untersuchende Medium ist. Das zu untersuchende Medium kann dann besser mit den an der Sensoroberfläche adhärent angelagerten Target- oder Rezeptorzellen in Verbindung gebracht werden, da es einfach in das Aufnahmebehältnis eingefüllt werden kann und dann gleich mit den Target- oder Rezeptorzellen in Berührung gerät.

Besonders vorteilhaft ist, wenn sich die Meßstruktur am Boden des Aufnahmebehältnisses befindet und wenn die Seitenwände durch eine den Meßbereich der Meßstruktur umgrenzende Verkapselung als seitliche Begrenzung ausgebildet sind. Der Sensor ist also so in die Wandung des Aufnahmebehältnisses eingebaut, daß nur der Meßbereich der Meßstruktur frei liegt, während die elektrischen Anschlüsse des Sensors durch die Verkapselung gegen das in dem Aufnahmebehältnis befindliche Medium abgedichtet sind.

Eine bevorzugte Ausführungsform der Erfindung sieht vor, daß mehrere Meßstrukturen vorzugsweise nebeneinander angeordnet sind. Dadurch können an verschiedenen Stellen eines zu untersuchenden Mediums gleichzeitig Messungen durchgeführt werden, so daß einerseits Inhomogenitäten in dem zu untersuchenden Medium festgestellt werden können und andererseits aber auch durch Vergleich der Meßsignale mehrere, benachbart zueinander angeordnete Meßstrukturen eine Kontrolle der Meßergebnisse möglich ist.

Vorteilhaft ist, wenn die elektrischen Kontaktstellen und/oder die strukturierten Zwischenschichten von wenigstens zwei Meßstrukturen unterschiedlicher Spezifität aufweisen. Die einzelnen Meßstrukturen ermöglichen dadurch eine selektive Messung unterschiedlicher Ionen, Moleküle oder Zellbereiche. Insbesondere kann auch das Abscheiden eines bestimmten Stoffes einer physiologsichen Substanz zum Beispiel bei bestimmten Zellprozessen selektiv gemessen werden. Als abgeschiedene Stoffe kommen beispielsweise CO, CO₂, NH₃, H₂S, CH₄, C₂H₅OH, O₂ NO sowie Protonen in Betracht.

Nachfolgend ist die Erfindung anhand von Ausführungsbeispielen näher erläutert.

Es zeigen zum Teil stärker schematisiert:
- Fig. 1: einen auf einem Substrat befindlichen Interdigitalkondensator mit mäanderförmig parallel zueinander verlaufenden Elektroden,
- Fig. 2: einen auf einem Substrat befindlichen Interdigitalkondensator mit kammartig ineinandergreifenden Kammelektroden,
- Fig. 3: eine dreidimensionale Darstellung der Elektroden eines Interdigitalkondensators,
- Fig. 4: einen Querschnitt durch das Substrat ohne die darauf befindlichen Rezeptorzellen, wobei als Meßstruktur ein Interdigitalkondensator vorgesehen ist und wobei auch das den Meßbereich umgrenzende Aufnahmebehältnis erkennbar ist,
- Fig. 5: eine Darstellung ähnlich Fig.4, wobei jedoch zusätzlich auch die an der Meßstruktur unmittelbar angelagerten Rezeptorzellen und der in das Aufnahmebehältnis eingefüllte Analyt gezeigt sind,
- Fig. 6: eine Aufsicht auf den Gatebereich eines ISFET'S mit Reizelektroden,
- Fig. 7: einen Querschnitt durch den in Fig.6 gezeigten ISFET, mit einer auf dem Gate befindlichen Targetzelle,
- Fig. 8: eine Teilansicht der Meßeinrichtung, die einen ISFET mit einer Zwischenschicht zeigt, mit der eine Rezeptorzelle in unmittelbarem Kontakt steht, wobei der Meßbereich des ISFET seitlich durch ein Aufnahmebehältnis begrenzt ist, das einen Analyten enthält,
- Fig. 9: eine Darstellung ähnlich Fig.8, wobei jedoch die Rezeptorzelle in einer eine Gelmembran aufweisenden Gelschicht angeordnet ist,
- Fig. 10: eine Aufsicht auf ein Halbleitersubstrat in das vier ISFETS, vier Referenz-FETS und eine Temperaturdiode integriert sind,
- Fig. 11: eine Aufsicht auf ein Halbleitersubstrat mit vier ISFETS, zwischen denen eine Referenzelektrode angeordnet ist, sowie mit zwei Referenz-FETS und einer Temperaturdiode,
- Fig. 12: eine Aufsicht auf ein Halbleitersubstrat mit einem aus ISFETS und Interdigitalkondensatoren bestehenden Sensor-Array, wobei auf dem Halbleitersubstrat auch ein Mikroprozessor mit AD/DA-Wandler, Multiplexer und IO-Einheit vorgesehen ist,
- Fig. 13: das Blockschaltbild des in Fig.12 gezeigten Biosensor-Chips,

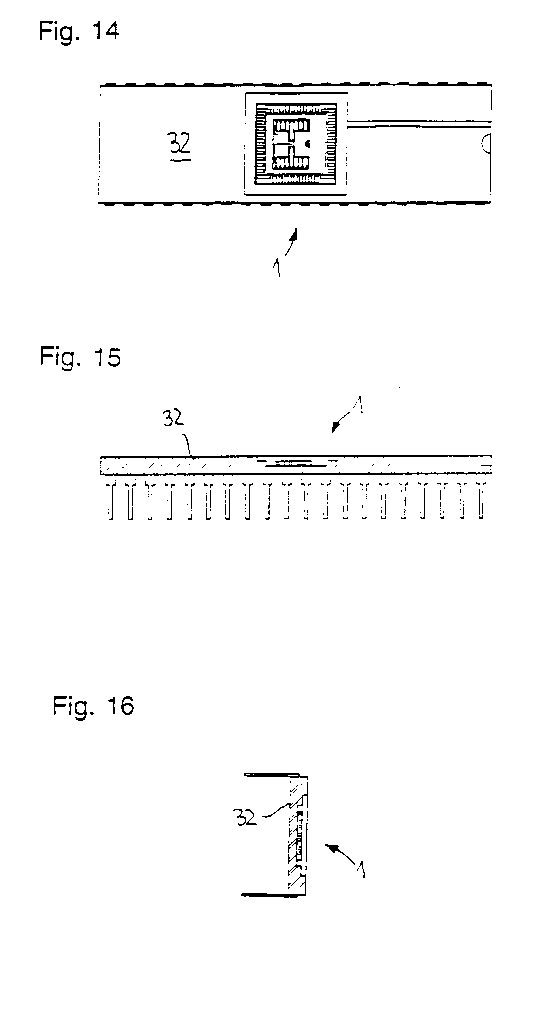

- Fig. 14: eine Aufsicht auf eine in ein DIL-Gehäuse eingebaute Meßeinrichtung,
- Fig. 15: einen Schnitt durch die Längsmittelebene der in Fig.14 dargestellten Meßeinrichtung,
- Fig. 16: einen Schnitt durch die Quermittelebene der in Fig. 14 gezeigten Meßeinrichtung,
- Fig. 17: eine Aufsicht auf eine in ein DIL-Gehäuse eingebaute Meßeinrichtung mit einem Aufnahmebehälter für den Analyten,
- Fig. 18: eine Längsseitenansicht der in Fig.17 gezeigten Meßeinrichtung, wobei das Aufnahmebehältnis und die Anschlußkontakte des DIL-Gehäuses besonders gut erkennbar sind,
- Fig. 19: eine Seitenansicht auf die Schmalseite der in Fig.17 und 18 gezeigten Meßeinrichtung,

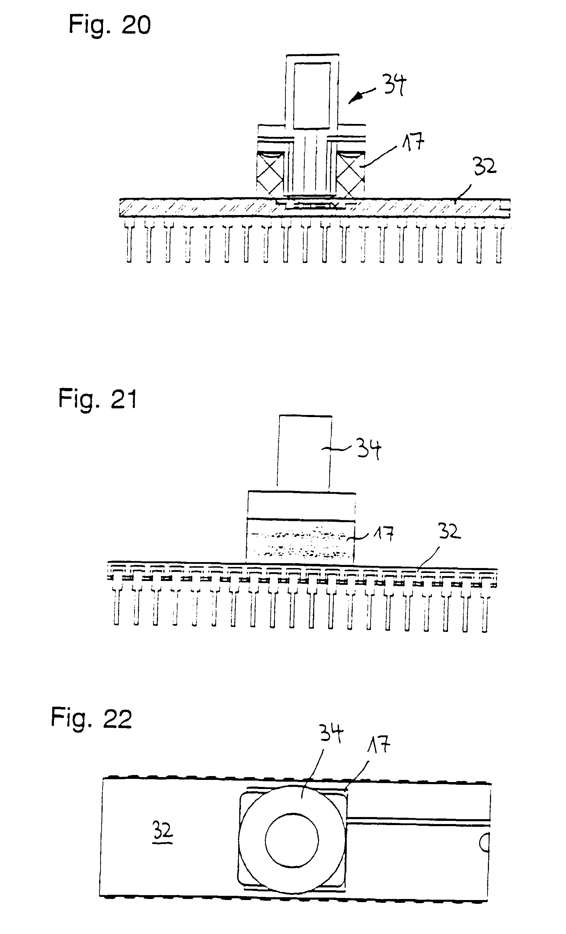

- Fig. 20: einen Schnitt durch die Längsmittelebene einer Meßeinrichtung ähnlich der in Fig.17, wobei jedoch auf die Einfüllöffnung des Aufnahmebehältnisses ein Durchflußaufsatz mit integrierter Referenzelektrode aufgesetzt ist,
- Fig. 21: eine Längsseitenansicht der in Fig. 20 gezeigten Meßeinrichtung und
- Fig. 22: eine Aufsicht auf die in Fig. 20 gezeigten Meßeinrichtung.

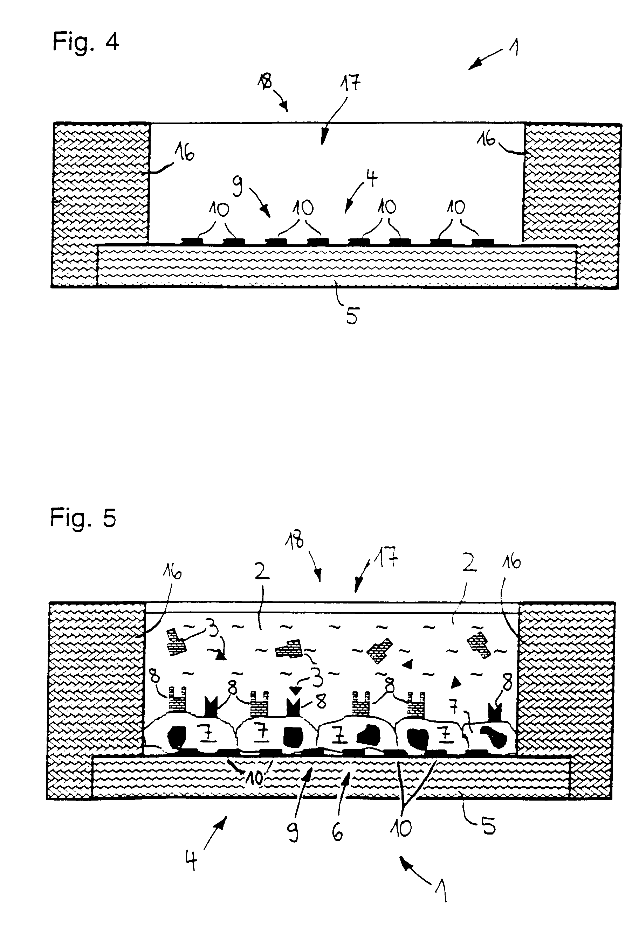

Eine im ganzen mit 1 bezeichnete Meßeinrichtung zur Messung oder Untersuchung physiologischer Parameter an biologischen Zellen oder von in einem Analyten 2 enthaltenen chemischen Komponenten 3 weist einen Sensor 4 mit einer auf einem Substrat 5 angeordneten Meßstruktur 6 auf (Fig. 5). Der Sensor 4 ist mit etwa 30 Rezeptorzellen 7 kombiniert, die an der Oberfläche der Meßstruktur 6 angeordnet sind und mit dieser unmittelbar in Kontakt stehen. Die Rezeptorzellen 7 weisen Rezeptoren 8 für ganz bestimmte chemische oder biologische Komponenten 3 auf. Wenn diese Komponenten 3 in dem Analyten 2 enthalten sind, lagern sie sich an den Rezeptoren 8 an. Die Komponenten 3 können beispielsweise Hormone, Antikörper, Antigene oder Wachstumsfaktoren sein. Die Rezeptorzellen 7 werden durch das Ankoppeln der chemischen Komponenten 3 verändert und diese Veränderung kann mit der Meßstruktur 6 detektiert werden. So bewirken beispielsweise bestimmte, in dem Analyten 2 enthaltene Schwermetallionen Formveränderungen der Rezeptorzellen 7, die in einer mit der Meßstruktur 6 detektierbaren Impedanzänderung der Zellmembran resultieren. Bei der erfindungsgemäßen Meßeinrichtung 1 mißt also der Sensor 4 Veränderungen der Rezeptorzellen 7, die durch die in dem Analyten 2 enthaltenen chemischen oder biologischen Komponenten 3 verursacht sind. Es handelt sich also hier praktisch um einen bio-elektronischen Sensor, bei dem ein biologischer Sensor (Rezeptor- oder Targetzellen) mit einem elektrischen oder elektronischen Sensor kombiniert ist.

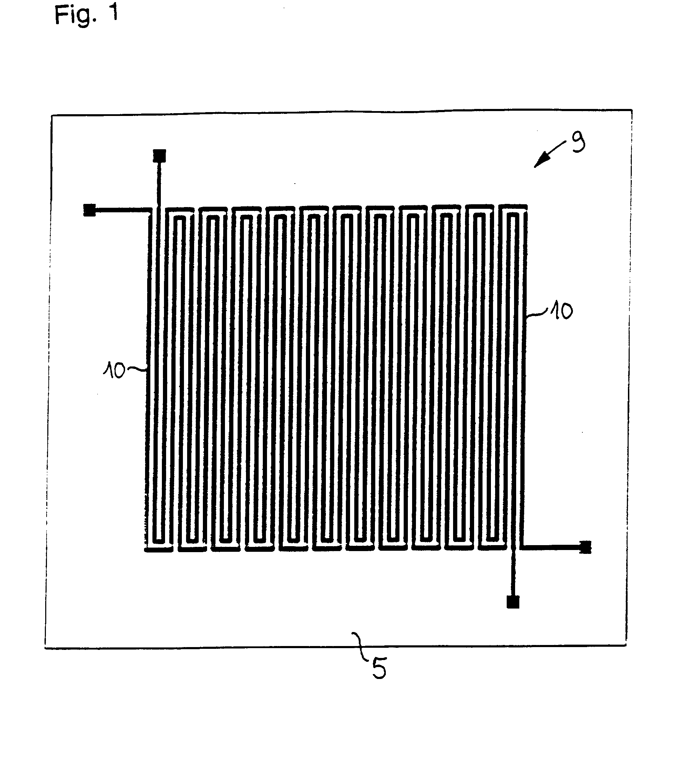

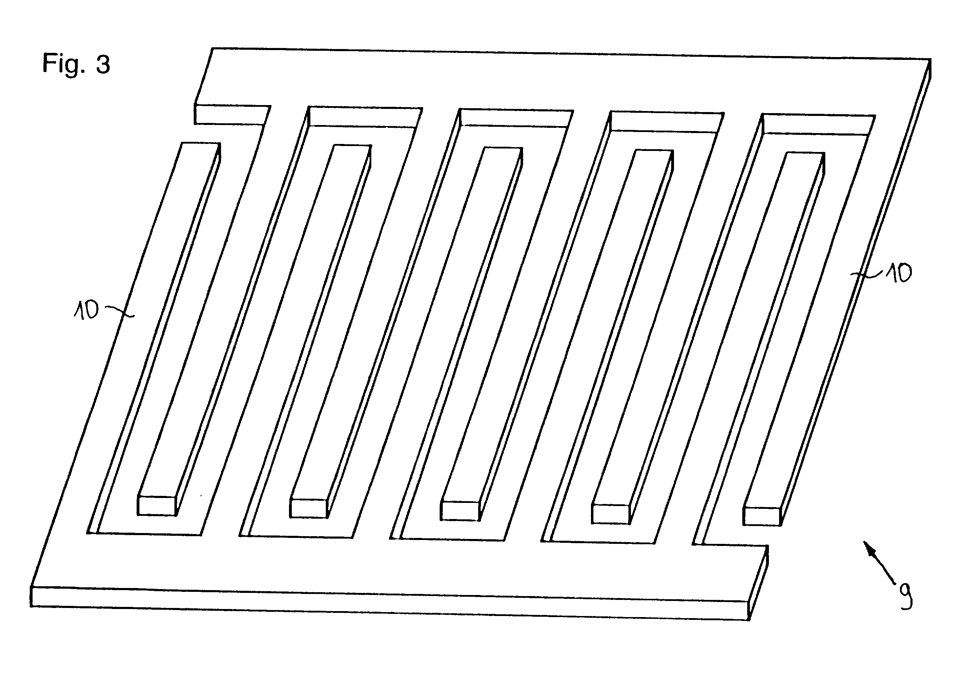

Bei der in Figur 5 gezeigten Ausführungsform ist als Meßstruktur 6 ein Interdigitalkondensator 9 vorgesehen. Die Elektroden 10 des Interdigitalkondensators 9 sind durch einander benachbarte Leiterbahnen gebildet, die auf einem ebenen Substrat 5, das beispielsweise aus Glas, Saphir oder Silizium bestehen kann angeordnet sind. Die Figuren 1 bis 3 zeigen Ausführungsbeispiele von Interdigitalkondensatoren 9 mit unterschiedlichem Elektroden-Layout. Bei dem Beispiel nach Figur 1 sind die Elektroden 10 durch zwei äquidistant zueinander verlaufende Leiterbahnen gebildet, die mäanderförmig an der Oberfläche des Substrats 5 angeordnet sind. Bei einer anderen Ausführungsform (Figur 2 und 3) weist der Interdigitalkondensator 9 zwei ineinandergreifende Kammelektroden auf. Mit dem Interdigitalkondensator 9 können sowohl Formveränderungen der Target- oder Rezeptorzellen 7 als auch Impedanzveränderungen an der Zellmembran dieser Zellen gemessen werden. Der Interdigitalkondensator 9 ermöglicht durch seinen planaren Aufbau eine besonders kostengünstig und in Großserie herstellbare Meßeinrichtung 1. Vorteilhaft ist dabei vor allem, daß die Elektrodenstruktur des Interdigitalkondensators 9 auch auf biokompatible Substrate aufgebracht werden kann.

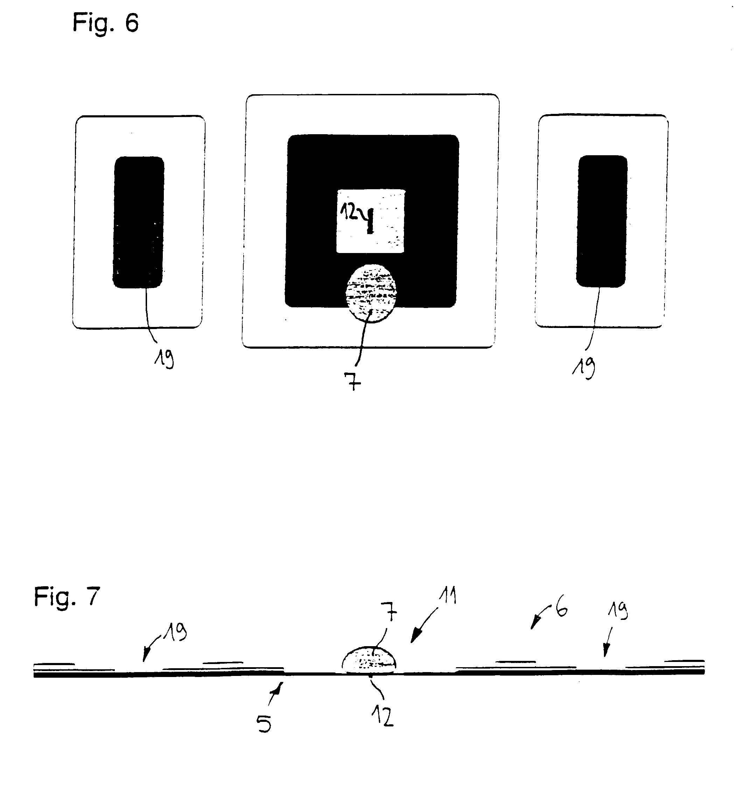

Die Figuren 6 bis 9 zeigen Ausführungsbeispiele der Meßeinrichtung 1, bei denen als Sensor ein ISFET 11 vorgesehen ist, dessen Gate 12 zum Kontakt mit den Rezeptorzellen 7 frei liegt. Zwischen dem für die Messung aktiven Bereich des ISFET's 11 und den Rezeptorzellen 7 ist eine Zwischenschicht 13 vorgesehen, die mit ihrer einen Flachseite mit dem Gate 12 und mit ihrer anderen Flachseite mit den Rezeptorzellen 7 direkt in Berührung steht. Die Zwischenschicht 13 ist eine makromolekulare poröse Schicht, die einerseits die Rezeptorzelle 7 zur Adhäsion bringt und andererseits in ihrer Porengröße so bemessen ist, daß sie für bestimmte Ionen, Moleküle oder Zellbereiche durchlässig ist. Die Zwischenschicht 13 ist also biokompatibel, so daß die Rezeptorzellen 7 diese als Nachbar akzeptieren und an ihr anhaften. Dennoch wird die Messung durch die zwischen der Meßstruktur 6 und den Rezeptorzellen 7 befindliche Zwischenschicht 13 nicht behindert, da diese für die zu detektierenden Moleküle, Ionen oder Zellbereiche durchlässig ist.

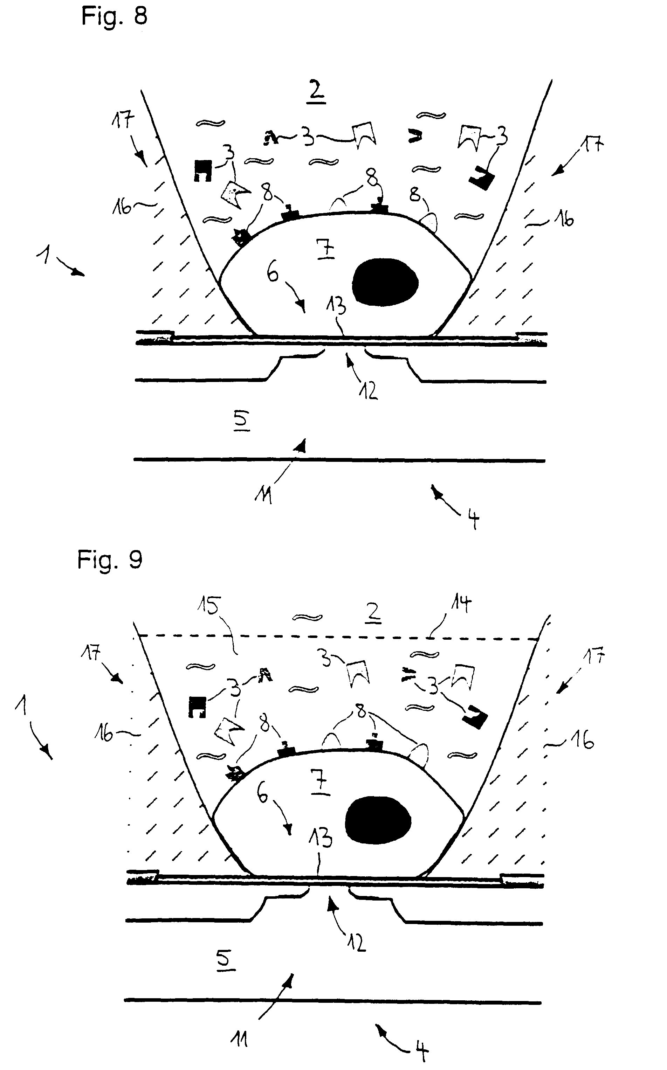

Bei dem in Figur 9 gezeigten Ausführungsbeispiel sind die Target- oder Rezeptorzellen 7 in einer eine Membran 14 aufweisenden Gelstruktur 15 angeordnet. Die Gelstruktur 15 beinhaltet ein Nährmedium und dient somit den Target- oder Rezeptorzellen 7 als Nahrungsspeicher. Außerdem ist in der Gelstruktur 15 Feuchtigkeit gespeichert, so daß die Target- oder Rezeptorzellen 7 über einen längeren Zeitraum am Leben erhalten werden können. Die Meßeinrichtung 1 ermöglicht deshalb auch über längere Zeitspannen hinweg eine kontinuierliche online-Analyse bestimmter, in dem Analyten 2 enthaltener Komponenten 3. Dabei sind die Gelstruktur 15 und die Membran 14 für die zu detektierenden Komponenten 3 durchlässig, so daß diese bis zu den Rezeptoren 8 der Rezeptorzellen 7 gelangen können.

Bei dem in Figur 8 gezeigten Ausführungsbeispiel ist das Nährmedium für die Rezeptorzellen 7 in dem Analyten 2 enthalten, so daß auch hier über einen längeren Zeitraum eine on-line-Signalgewinnung möglich ist.

Wie aus den Figuren 4, 5, 8 und 9 besonders gut erkennbar ist, ist die Meßstruktur 6 Teil einer Wandung 16 eines Aufnahmebehältnisses 17. Dabei sind die Meßstruktur 6 und die darauf befindlichen Target- oder Rezeptorzellen 7 am Boden des Aufnahmebehälters 17 angeordnet, so daß der Analyt 2 auf einfache Weise in das Aufnahmebehältnis 17 eingefüllt werden kann und dann gleich mit den Target- oder Rezeptorzellen 7 in Berührung gerät. Die Seiten-Wandungen 16 des Aufnahmebehältnisses 17 sind dicht mit dem Substrat 5 verbunden und umgrenzen den für die Messung vorgesehenen Bereich 6. Dadurch wird auch bei einer größeren Menge eines zu analysierenden Mediums verhindert, daß dieses seitlich ausfließt und dabei mit dafür nicht vorgesehenen Bereichen des Sensors 4, insbesondere mit dessen Anschlußkontakten in Berührung gerät.

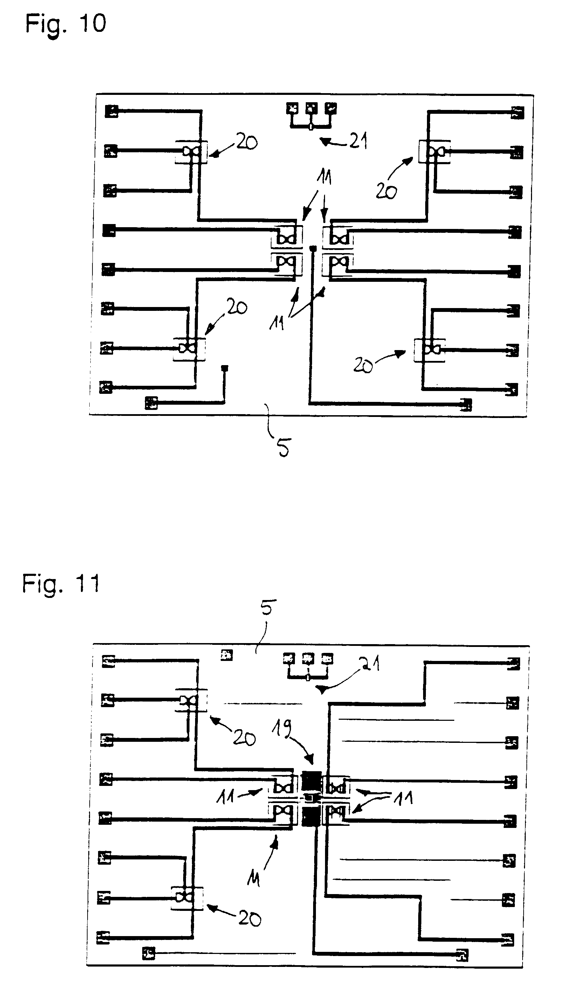

Bei den in Figur 6 und 7 gezeigten Substraten 5 ist beidseits eines ISFET's 11 jeweils eine Reizelektrode 19 vorgesehen, mit der die Rezeptorzellen 7 zur spontanen Abgabe einer zu detektierenden Substanz angeregt werden können. Besonders vorteilhaft ist, wenn mehrere Sensoren 4 auf einem gemeinsamen Substrat 5 angeordnet sind. Figur 10 und 11 zeigt dies beispielhaft an einem Substrat 5 mit vier zum Kontakt mit Target- oder Rezeptorzellen 7 vorgesehenen ISFET's 11, wobei zusätzlich noch vier weitere als Referenzelemente 20 dienende ISFET's außerhalb des für den Bewuchs mit den Target- oder Testzellen vorgesehenen Substratbereich in das Substrat 5 integriert sind. Die Referenzelemente 20 ermöglichen eine Kompensation der Temperaturdrift und der Offset-Spannung der Sensoren 4. Bedarfsweise können benachbart zu den Sensoren 4 noch Reizelektroden 19 vorgesehen sein (Fig. 11).

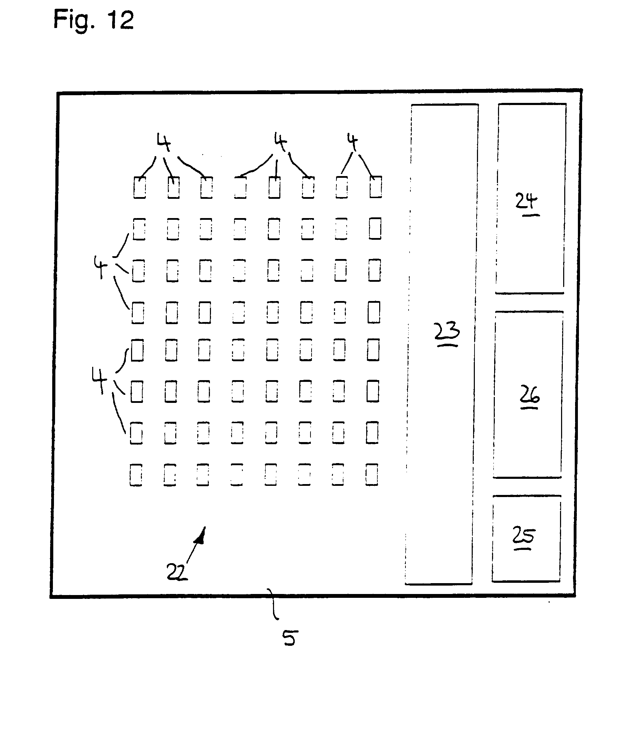

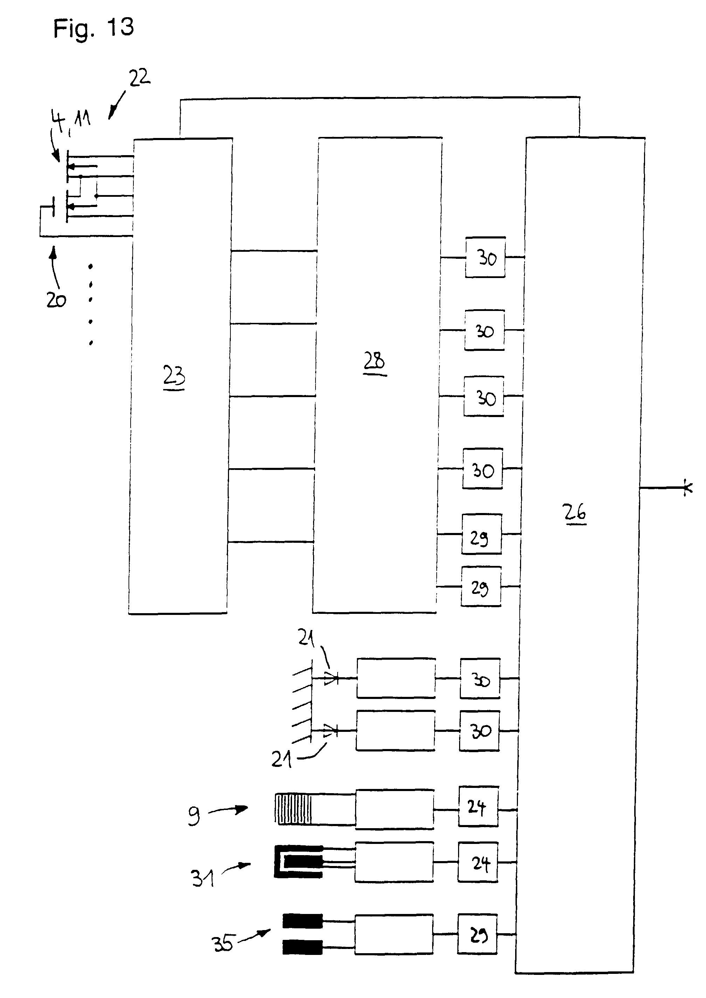

Besonders vorteilhaft ist, wenn die Sensoren 4 in Form eines Arrays auf dem Substrat 5 angeordnet sind (Fig. 12). Mit einer einzigen Meßeinrichtung 1 können dann mehrere, unterschiedliche chemische oder biologische Komponenten 3 gleichzeitig detektiert werden. Außerdem besteht die Möglichkeit einer parallelen Meßsignalgewinnung an unterschiedlichen Target- oder Rezeptorzellen, so daß eine Kontrolle der Meßergebnisse möglich ist. Bei dem in Figur 12 gezeigten Ausführungsbeispiel sind zusätzlich zu dem Sensor-Array 22 noch ein Multiplexer 23, eine AD/DA-Wandlereinheit 24, eine IO-Einheit 25 und ein Mikroprozessor 26 integriert. Wie aus dem zugehörigen Blockschaltbild (Figur 13) erkennbar ist, können beliebige, auf dem Substrat 5 angeordnete Sensoren jeweils einer von fünf gleichzeitig ansteuerbaren Meßleitungen 27 zugeordnet sein. Eine Regelungseinheit 28 ermöglicht dabei eine Einstellung der für die ausgewählten Sensoren 4 und den denen zugeordneten Referentelementen 20 vorgesehenen Strom- und Spannungswerte. Die entsprechenden Werte werden von dem Mikroprozessor 26 ermittelt und über Digital/Analog-Wandler 29 an die Regelungseinheit 28 ausgegeben. Zum Einlesen der Meßwerte aus der Regelungseinheit 28 sind Analog/Digital-Wandler 30 vorgesehen. An dem Mikroprozessor sind außerdem zwei Temperatursensoren 21, ein Interdigitalkondensator 9, ein Sauerstoffsensor 31 sowie zwei AgCl-Referenzelektroden 35 angeschlossen.

Das komplette Substrat 5 mit den darauf befindlichen Target- oder Rezeptorzellen 7 ist in einen 40-poligen Keramik-DIL-Sockel 32 eingebaut. Die Meßeinrichtung 1 kann dadurch wie eine handelsübliche integrierte Schaltung beispielsweise in einen auf einer Platine vorgesehenen IC-Sockel eingesetzt werden.

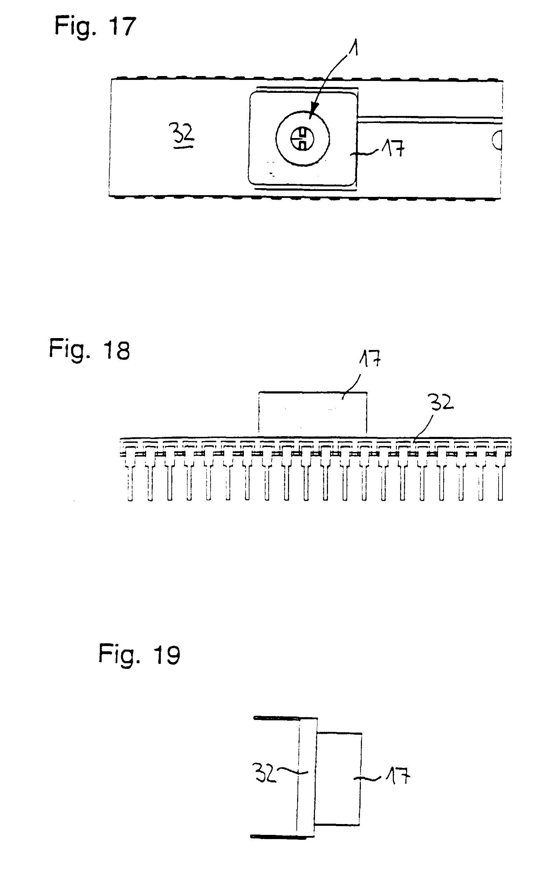

Die Figuren 17 bis 19 zeigen Ausführungsbeispiele, bei denen auf dem Keramik-DIL-Sockel 32 ein Aufnahmebehältnis 17 für den Analyten 2 vorgesehen ist. Der Analyt 2 kann dann auf einfache Weise in das Aufnahmebehältnis 17 eingefüllt werden. Um eine besonders exakte Positionierung des Analyten 2 in Bezug zu den Sensoren 4 zu ermöglichen, kann in die Einfüllöffnung 18 des Aufnahmebehältnisses 17 ein Durchflußaufsatz 34 eingesetzt werden. Diffusionsunterschiede des Analyten 2, wie sie beispielsweise bei dessen Einfüllen oder Absaugen mit einer Pipette im Bereich des Sensors 4 auftreten können, werden dadurch weitgehend vermieden.

## Patentansprüche

1. Meßeinrichtung (1) zur Messung oder Untersuchung physiologischer Parameter an biologischen Zellen oder von in einem Analyten (2) enthaltenen chemischen und bioaktiven Substanzen (3), mit wenigstens einem elektrischen oder elektronischen Sensor (4), der einen mit einer Auswerteeinrichtung verbindbaren Meßausgang aufweist, wobei der Sensor (4) wenigstens eine auf einem Substrat (5) befindliche, elektrische oder elektronische Meßstruktur (6) aufweist, die mit funktionsspezifischen, als biologischer Sensor (Diskriminator) dienenden Rezeptorzellen (7) und/oder Targetzellen verbunden ist und zusammen mit diesen Zellen einen Sensor bilden, wobei die Zellen inhärenter Bestandteil des Sensors sind und der zu untersuchende Analyt (2) mit den Rezeptorzellen (7) und/oder den Targetzellen auf derer der Meßstruktur (6) abgewandten Seite zur Messung in Verbindung bringbar ist, **dadurch gekennzeichnet**, daß zwischen den Rezeptor- und/oder Targetzellen und der Meßstruktur (6) eine strukturierte, mikroporöse Zwischenschicht (13) vorgesehen ist, welche die Rezeptor- und/oder Targetzellen als Nachbarn zur Adhärenz akzeptiert.

2. Meßeinrichtung nach Anspruch 1, dadurch gekennzeichnet, daß auf einem gemeinsamen Substrat (5) mehrere Sensoren (4) mit Meßstrukturen (6) insbesondere als Sensorarray (22) angeordnet sind.

3. Meßeinrichtung nach Anspruch 2, dadurch gekennzeichnet, daß auf einem gemeinsamen Substrat (5) mehrere Sensoren (4) mit gleichen Meßstrukturen (6) insbesondere als Sensorarray (22) angeordnet sind.

4. Meßeinrichtung nach Anspruch 2 oder 3, dadurch gekennzeichnet, daß auf einem gemeinsamen Substrat (5) mehrere Sensoren (4) mit unterschiedlichen Meßstrukturen (6) insbesondere als Sensorarray (22) angeordnet sind.

5. Meßeinrichtung nach einem der Ansprüche 2 bis 4, dadurch gekennzeichnet, daß auf einem gemeinsamen Substrat (5) mehrere zellbehaftete Meßstrukturen (6) und zusätzlich zellfreie Meßstrukturen vorgesehen sind.

6. Meßeinrichtung nach einem der Ansprüche 1 bis 5, dadurch gekennzeichnet, daß die Meßausgänge der auf einem gemeinsamen Substrat (5) angeordneten Sensoren (4) mit einer insbesondere auf dem Substrat (5) integriert angeordneten Steuer- und Auswerteeinrichtung verbunden sind.

7. Meßeinrichtung nach Anspruch 6, dadurch gekennzeichnet, daß sich auf dem Substrat (5) mit dem Sensorarray (22) als Steuer- und Auswerteeinrichtung ein Multiplexer (23), ein AD/DA-Wandler (24) mit Sensoransteuerung, ein Mikroprozessor (26) sowie eine IO-Einheit (25) befinden.

8. Meßeinrichtung nach einem der Ansprüche 1 bis 7, dadurch gekennzeichnet, daß auf dem die Meßstruktur (6) aufweisenden Substrat (5) innerhalb des Meßbereiches oder benachbart zu diesem wenigstens eine Reizelektrode (19) für die Rezeptor- oder Targetzellen und/oder eine Referenzelektrode angeordnet ist.

9. Meßeinrichtung nach einem der Ansprüche 1 bis 8, dadurch gekennzeichnet, daß das Substrat (5) ein Halbleitersubstrat ist und daß als Meßstruktur wenigstens ein Feldeffekttransistor, insbesondere ein ISFET (11) vorgesehen ist, dessen Gate (12) zum Kontakt mit den Rezeptor- oder Targetzellen freiliegt.

10. Meßeinrichtung nach einem der Ansprüche 1 bis 9, dadurch gekennzeichnet, daß als Meßstruktur (6) wenigstens ein Interdigitalkondensator (9), dervorzugsweisepaarig ineinandergreifende Elektroden (10) aufweist, vorgesehen ist.

11. Meßeinrichtung nach Anspruch 10, dadurch gekennzeichnet, daß mehrere, vorzugsweise unterschiedlich große Interdigitalkondensatoren (9) vorgesehen sind.

12. Meßeinrichtung nach Anspruch 10 oder 11, dadurch gekennzeichnet, daß in wenigstens einem isolierten Zwischenraum der Elektroden (10) des Interdigitalkondensators (9) eine elektrochemosensitive Schicht vorgesehen ist.

13. Meßeinrichtung nach einem der Ansprüche 10 bis 12, dadurch gekennzeichnet, daß zwischen den Elektroden (10) des Interdigitalkondensators (9) Lichtleiter vorgesehen sind und daß Lichtdetektoren zur Aufnahme und zum Nachweis des den jeweiligen Lichtleiter durchlaufenden Lichts im Substrat (5) angeordnet sind.

14. Meßeinrichtung nach einem der Ansprüche 1 bis 13, dadurch gekennzeichnet, daß in das Substrat (5) CCD-Sensoren, insbesondere in Form einer CCD-Zeile oder eines CCD-Arrays integriert sind.

15. Meßeinrichtung nach einem der Ansprüche 1 bis 14, dadurch gekennzeichnet, daß auf dem die Meßstruktur (6) aufweisenden Substrat (5) außerhalb des für die Messung vorgesehenen Substratbereiches wenigstens ein Referenzelement (20) mit gleichem oder ähnlichem Aufbau wie dem der Meßstruktur (6) angeordnet ist, insbesondere bei einem Feldeffekttransistor als Meßstruktur ein Feldeffekttransistor mit Gate.

16. Meßeinrichtung nach einem der Ansprüche 1 bis 15, dadurch gekennzeichnet, daß auf dem die Meßstruktur (6) aufweisenden Substrat (5) wenigstens ein Temperaturmeßsensor (21), insbesondere eine Temperaturmeßdiode angeordnet ist.

17. Meßeinrichtung nach einem der Ansprüche 1 bis 16, dadurch gekennzeichnet, daß die Rezeptorzellen (7) in einer Gelstruktur (15) angeordnet oder von dieser überdeckt sind.

18. Meßeinrichtung nach einem der Ansprüche 1 bis 17, dadurch gekennzeichnet, daß die elektrische oder elektronische Meßstruktur (6) Teil einer Wandung (16) eines Aufnahmebehältnisses (17) für das zu untersuchende Medium ist.

19. Meßeinrichtung nach Anspruch 18, dadurch gekennzeichnet, daß sich die Meßstruktur (6) am Boden des Aufnahmebehältnisses (17) befindet und daß die Seitenwände durch eine den Meßbereich der Meßstruktur (6) umgrenzende Verkapselung als seitliche Begrenzung gebildet sind.

20. Meßeinrichtung nach einem der Ansprüche 1 bis 19, dadurch gekennzeichnet, daß mehrere Meßstrukturen (6) vorzugsweise nebeneinander angeordnet sind.

21. Meßeinrichtung nach einem der Ansprüche 1 bis 20, dadurch gekennzeichnet, daß die elektrischen Kontaktstellen und/oder die strukturierten Zwischenschichten (13) von wenigstens zwei Meßstrukturen (6) unterschiedliche Spezifitäten aufweisen.

## Claims

1. A measuring apparatus (1) for measurement or examination of physiological parameters of biological cells or chemical and bioactive substances (3) contained in an analyte (2), with at least one electrical or electronic sensor (4) which has a measurement output which can be connected to an evaluation apparatus, wherein the sensor (4) has at least one electrical or electronic measurement structure (6) located on a substrate (5) which is connected to function-specific receptor cells (7) which serve as biological sensors (discriminators) and/or target cells and which forms a sensor together with these cells, wherein the cells are an inherent component of the sensor, and the analyte (2) to be examined can be connected to the receptor cells (7) and/or the target cells on the side remote to the measurement structure (6) for the purposes of measurement, characterised in that a structured microporous intermediate layer (13) is provided between the receptor and/or target cells and the measurement structure (6) which accepts the receptor and/or target cells as neighbours for adherence.

2. A measuring apparatus according to claim 1, characterised in that a plurality of sensors (4) with measurement structures (6) are disposed on a common substrate (5), especially as a sensor array (22).

3. A measuring apparatus according to claim 2, characterised in that a plurality of sensors (4) with identical measurement structures (6) are disposed on a common substrate (5), especially as a sensor array (22).

4. A measuring apparatus according to claim 2 or 3, characterised in that a plurality of sensors (4) with different measurement structures (6) are disposed on a common substrate (5), especially as a sensor array (22).

5. A measuring apparatus according to one of claims 2 to 4, characterised in that a plurality of cell-filled measuring structures (6) and additional cell-free measurement structures are provided on a common substrate (5).

6. A measuring apparatus according to one of claims 1 to 5, characterised in that the measurement outputs of the sensors (4) disposed on a common substrate (5) are connected to a control and evaluation apparatus, especially integrated on the substrate (5).

7. A measuring apparatus according to claim 6, characterised in that a multiplexer (23), an AD/DA converter (24) with sensor control, a microprocessor (26) and an IO unit (25) are disposed on the substrate (5) with the sensor array (22) as a control and evaluation apparatus.

8. A measuring apparatus according to one of claims 1 to 7, characterised in that at least one stimulation electrode (19) for the receptor or target cells and/or a reference electrode is disposed on the substrate (5) which carries the measurement structure (6), within the measurement range or adjacent thereto.

9. A measuring apparatus according to one of claims 1 to 8, characterised in that the substrate (5) is a semiconductor substrate and that at least one field effect transistor, especially an ISFET (11) is provided as a measurement structure, whose gate (12) is available for contact with the receptor or target cells.

10. A measuring apparatus according to one of claims 1 to 9, characterised in that at least one inter-digital condenser (9), which preferably has electrodes (10) interlocking in pairs, is provided as a measuring structure (6).

11. A measuring apparatus according to claim 10, characterised in that a plurality of inter-digital condensers (9), preferably of different sizes, are provided.

12. A measuring apparatus according to claim 10 or 11, characterised in that an electrochemosensitive layer is provided in at least one isolated intermediate space of the electrodes (10) of the inter-digital condenser (9).

13. A measuring apparatus according to one of claims 10 to 12, characterised in that light conductors are provided between the electrodes (10) of the inter-digital condenser (9) and that light detectors are disposed in the substrate (5) to record and detect the light passing through the respective light conductors.

14. A measuring apparatus according to one of claims 1 to 13, characterised in that CCD sensors, especially in the form of a CCD line or a CCD array are integrated in the substrate (5).

15. A measuring apparatus according to one of claims 1 to 14, characterised in that at least one reference element (20) with an identical or similar construction to the measurement structure (6) is disposed on the substrate (5) which carries the measurement structure (6) outside of the substrate area which has been provided for the measurement, especially a field effect transistor with a gate where a field effector transistor has been provided as a measurement structure.

16. A measuring apparatus according to one of claims 1 to 15, characterised in that at least one temperature measurement sensor (21), especially a temperature measurement diode, is disposed on the substrate (5) which carries the measurement structure (6).

17. A measuring apparatus according to one of claims 1 to 16, characterised in that the receptor cells (7) are disposed in a gel structure (15) or are covered by this.

18. A measuring apparatus according to one of claims 1 to 17, characterised in that the electrical or electronic measurement structure (6) is part of a wall (16) of a reception container (17) for the medium which is to be examined.

19. A measuring apparatus according to claim 18, characterised in that the measurement structure (6) is disposed on the base of the reception container (17) and that the side walls are formed as a lateral boundary by an encapsulation which defines the measurement area of the measurement structure (6).

20. A measuring apparatus according to one of claims 1 to 19, characterised in that a plurality of measurement structures (6) are preferably disposed adjacent to one another.

21. A measuring apparatus according to one of claims 1 to 20, characterised in that electrical contact points and/or the structured intermediate layers (13) of at least two measurement structures (6) have different specificities.

## Revendications

1. Dispositif de mesure (1) conçu pour la mesure ou l'examen de paramètres physiologiques sur des cellules biologiques, ou de substances chimiques et bioactives (3) renfermées par un objet d'analyse (2), comprenant au moins un capteur (4) électrique ou électronique qui présente une sortie de mesure pouvant être raccordée à un dispositif d'interprétation, ledit capteur (4) comportant au moins une structure de mesure (6) électrique ou électronique située sur un substrat (5), qui est reliée à des cellules réceptrices (7) à spécificité fonctionnelle, servant de capteur biologique (discriminateur), et/ou à des cellules cibles, et qui forme un capteur en association avec ces cellules, lesdites cellules faisant partie intégrante du capteur, et l'objet d'analyse (2) à examiner pouvant être mis en liaison, en vue de la mesure, avec les cellules réceptrices (7) et/ou avec les cellules cibles, sur le côté desdites cellules tourné à l'opposé de la structure de mesure (6), caractérisé par le fait qu'il est prévu, entre les cellules réceptrices et/ou les cellules cibles et la structure de mesure (6), une couche intercalaire (13) microporeuse et structurée qui accepte lesdites cellules réceptrices et/ou lesdites cellules cibles en tant qu'élément limitrophe, en vue de l'adhérence.

2. Dispositif de mesure selon la revendication 1, caractérisé par le fait que plusieurs capteurs (4) munis de structures de mesure (6) sont placés sur un substrat commun (5), notamment en tant que rangée (22) de capteurs.

3. Dispositif de mesure selon la revendication 2, caractérisé par le fait que plusieurs capteurs (4) munis de structures de mesure identiques (6) sont placés sur un substrat commun (5), notamment en tant que rangée (22) de capteurs.

4. Dispositif de mesure selon la revendication 2 ou 3, caractérisé par le fait que plusieurs capteurs (4) munis de structures de mesure différentes (6) sont placés sur un substrat commun (5), notamment en tant que rangée (22) de capteurs.

5. Dispositif de mesure selon l'une des revendications 2 à 4, caractérisé par le fait que plusieurs structures de mesure (6) affectées à des cellules et, additionnellement, des structures de mesure exemptes de cellules, sont prévues sur un substrat commun (5).

6. Dispositif de mesure selon l'une des revendications 1 à 5, caractérisé par le fait que les sorties de mesure des capteurs (4), placés sur un substrat commun (5), sont raccordées à un dispositif de commande et d'interprétation occupant, en particulier, une position intégrée sur ledit substrat (5).

7. Dispositif de mesure selon la revendication 6, caractérisé par le fait qu'un multiplexeur (23), un convertisseur analogique-numérique/numérique-analogique (24) à activation par capteur, un microprocesseur (26), ainsi qu'une unité d'entrée/sortie (25), matérialisent le dispositif de commande et d'interprétation sur le substrat (5) présentant la rangée (22) de capteurs.

8. Dispositif de mesure selon l'une des revendications 1 à 7, caractérisé par le fait qu'au moins une électrode de stimulation (19) assignée aux cellules réceptrices ou aux cellules cibles, et/ou une électrode de référence, se trouve sur le substrat (5) présentant la structure de mesure (6), à l'intérieur de la plage de mesure ou au voisinage de celle-ci.

9. Dispositif de mesure selon l'une des revendications 1 à 8, caractérisé par le fait que le substrat (5) est un substrat semi-conducteur ; et par le fait qu'il est prévu, en tant que structure de mesure, au moins un transistor à effet de champ, en particulier un ISFET (11) dont la porte (12) est dégagée en vue du contact avec les cellules réceptrices ou les cellules cibles.

10. Dispositif de mesure selon l'une des revendications 1 à 9, caractérisé par le fait qu'il est prévu, en tant que structure de mesure (6), au moins un condensateur d'interdigitation (9) présentant, de préférence, des électrodes (10) à interpénétration appariée.

11. Dispositif de mesure selon la revendication 10, caractérisé par la présence de plusieurs condensateurs d'interdigitation (9), de tailles préférentiellement différentes.

12. Dispositif de mesure selon la revendication 10 ou 11, caractérisé par le fait qu'une couche électrochimiquement sensible est prévue dans au moins un espace intermédiaire isolé des électrodes (10) du condensateur d'interdigitation (9).

13. Dispositif de mesure selon l'une des revendications 10 à 12, caractérisé par le fait que des guides de lumière sont prévus entre les électrodes (10) du condensateur d'interdigitation (9) ; et par le fait que des détecteurs de lumière sont logés dans le substrat (5), en vue de la réception et de l'identification de la lumière parcourant le guide de lumière considéré.

14. Dispositif de mesure selon l'une des revendications 1 à 13, caractérisé par le fait que des capteurs CCD, revêtant notamment la forme d'une ligne CCD ou d'une rangée CCD, sont intégrés dans le substrat (5).

15. Dispositif de mesure selon l'une des revendications 1 à 14, caractérisé par le fait qu'au moins un élément de référence (20) se trouve sur le substrat (5) muni de la structure de mesure (6), à l'extérieur de la région dudit substrat qui est prévue pour la mesure, ledit élément présentant un agencement structurel identique ou analogue à celui de la structure de mesure (6), en particulier un transistor à effet de champ pourvu d'une porte lorsque ladite structure de mesure revêt la forme d'un transistor à effet de champ.

16. Dispositif de mesure selon l'une des revendications 1 à 15, caractérisé par le fait qu'au moins un capteur thermométrique (21), en particulier une diode thermométrique, se trouve sur le substrat (5) présentant la structure de mesure (6).

17. Dispositif de mesure selon l'une des revendications 1 à 16, caractérisé par le fait que les cellules réceptrices (7) sont logées dans une structure (15) constituée d'un gel, ou sont recouvertes par cette dernière.

18. Dispositif de mesure selon l'une des revendications 1 à 17, caractérisé par le fait que la structure de mesure électrique ou électronique (6) fait partie d'une paroi (16) d'un réceptacle (17) destiné au milieu à examiner.

19. Dispositif de mesure selon la revendication 18, caractérisé par le fait que la structure de mesure (6) se trouve au fond du réceptacle (17) ; et par le fait que les parois latérales sont formées par un encapsulage remplissant la fonction d'une délimitation latérale, qui circonscrit la plage de mesure de ladite structure de mesure (6).

20. Dispositif de mesure selon l'une des revendications 1 à 19, caractérisé par le fait que plusieurs structures de mesure (6) sont préférentiellement juxtaposées.

21. Dispositif de mesure selon l'une des revendications 1 à 20, caractérisé par le fait que les zones de contact électrique, et/ou les couches intercalaires structurées (13) d'au moins deux structures de mesure (6), présentent des spécificités différentes.
